# EUROPEAN PATENT APPLICATION

(11) **EP 0 966 973 A1**
(43) Date of publication of application: **29.12.1999**
(21) Application number: 97930838.4
(22) Date of filing: 18.07.1997
(51) Int. Cl.: A61K 47/30

(54) **SORBEFACIENTS**

(30) Priority: 23.07.1996 JP 19304596; 31.07.1996 JP 20257296
(71) Applicant: DAIICHI PHARMACEUTICAL CO., LTD., Chuo-ku, Tokyo 103 (JP)
(72) Inventor: NAKAGAMI, Hiroaki, Daiichi Phar. Co., Ltd., Edogawa-ku, Tokyo 134 (JP); YAMAO, Tadanao, Daiichi Phar. Co., Ltd., Edogawa-ku, Tokyo 134 (JP); FUJII, Yoshimine, Daiichi Phar. Co., Ltd., Edogawa-ku, Tokyo 134 (JP)
(74) Representative: Wächtershäuser, Günter, Prof. Dr.
(86) International application number: JP9702500
(87) International publication number: WO9803202

(57) **Abstract**

Pharmaceutical compositions for improving the absorption of drugs having poor absorbability in the digestive tract. The compositions contain drugs and anion exchange resins and show excellent absorbability in the digestive tract.

## Description

### Field of the Invention

The present invention relates to a pharmaceutical composition for improving absorption of drugs having poor absorbability when in the digestive tract administered perorally or in other manners.

### Background Art

Conventionally, among drugs which are poorly absorbed by the digestive tract, some have been known to not provide expected pharmacological effects due to their poor bioavailability.

These drugs having poor absorbability by the digestive tract are conventionally administered through intravenous or intramuscular injection. Administration through injection involves a variety of problems, such as inconvenience to use, in addition to pain and muscle disorder.

In order to solve these problems, there is proposed a method employing a promoting effect on absorption by use of a variety of additives (absorption enhancers). However, in some cases, when certain types of absorption enhancers are absorbed, they cause toxicity, leading to a problem for safety.

As mentioned above, conventional techniques provide no effective and safe method for improving absorption of drugs having poor absorbability by the digestive tract.

The present invention was made in an attempt to solve the above-described problems involved in the conventional techniques, and an object of the invention is to improve absorption of drugs having poor bioavailability in the digestive tract when administered perorally.

### Disclosure of the Invention

The present inventors have conducted earnest studies on solutions to the problems involved in drugs exhibiting poor bioavailability after peroral administration, and have found that the absorption of drugs in the digestive tract can be improved by addition of an anion-exchange resin which is widely used as a remover of impurity ions; a catalyst; and a drug such as a cholesterol lowering drug. The present invention has been accomplished based on this finding.

Accordingly, the present invention provides an absorption enhancer for drugs that contains an anion-exchange resin as an active component. The present invention also provides a method for promoting the absorption of drugs in the digestive tract by use of an anion-exchange resin. Furthermore, the present invention provides a pharmaceutical composition containing a drug and an anion-exchange resin.

### Best Modes for Carrying Out the Invention

In the present invention, the term "anion-exchange resin" refers to a water-insoluble synthetic resin having a basic ion-exchangeable group, which is hardly absorbed through the digestive tract membrane and exhibits no toxicity.

Examples of the basic ion-exchangeable group contained in the anion-exchange resin include an amino group, substituted amino groups (-NHR, -NRR'), quaternary ammonium groups (-N⁺RR'R''), an amidino group, aromatic amino groups (e.g., a pyridinyl group and a pyrrolidinyl group), and an alkyl group substituted with a group such as an amino group, a substituted amino group (-NHR, -NRR'), a quaternary ammonium group (-N⁺RR'R''), or an amidino group. More specific examples the basic ion-exchangeable group include an amino group, a methylamino group, -N⁺(CH₃)₃, -N⁺(CH₃)₂(CH₂CH₂OH), -CH₂-N⁺(CH₃)₃, and an imidazolium group.

In the present invention, the anion-exchange resins may be used singly or in combination of two or more species. No particular limitation is imposed on the resins so long as they have a group such as the above-described basic ion-exchangeable groups and can be administered to the human body. Preferable examples thereof include cholestyramin, colestipol hydrochloride, a compound having a structural unit represented by the following formula: 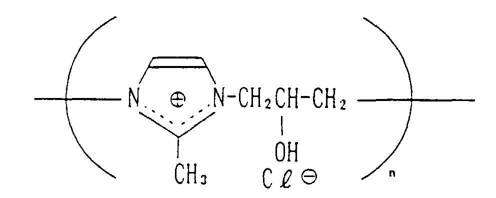 and a compound having a structural unit represented by the following formula.

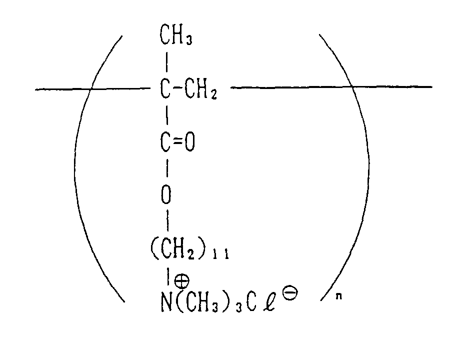

Of these, cholestyramin and compounds having either or both of the above-described structural units are particularly preferable anion-exchange resins in the present invention. Compounds having the above-described structural units may be homopolymers or copolymers having another structural unit.

The anion-exchange resins which meet the object of the present invention have characteristics as follows.

The average particle size, as measured by use of a laser diffraction-type particle size distribution-measuring apparatus, is 1 µm to 1 mm, preferably 10 µm to 500 µm.

The true density is 0.5-2.5 g/cm³, preferably 0.75-1.5 g/cm³.

The water content (weight of water/total weight) is 0.01-99.9%, preferably 0.1-80%.

The anion-exchange capacity is 1.0 mEq/g or more, preferably 1.5-6.0 mEq/g, particularly preferably 2.5-5.0 mEq/g.

In the present invention, the term "drug" encompasses extracts of a crude drug (extract, tincture, etc.) and compounds. The drugs may be used singly or in combination of two or more species. When the drug is a compound, there are included a salt thereof; a hydrate and pharmaceutically acceptable solvates thereof; a hydrate of the salt; and a solvate of the salt; as well as a crystalline polymorph of compounds. When the compound contains an asymmetric carbon atom in its structure and an optical isomer or a stereoisomer exists, the scope of the present invention also covers the optical isomer, the stereoisomer, and a mixture thereof.

No particular limitation is imposed on the salts of a compound so long as they are pharmaceutically acceptable. Specific examples include inorganic acid salts such as hydrochloride salts, hydrobromic acid salts, hydroiodic acid salts, phosphate salts, nitrate salts, and sulfate salts; organic sulfonate salts such as methanesulfonate salts, 2-hydroxyethanesulfonate salts, and p-toluenesulfonate salts; and organic carboxylate salts such as acetate salts, propionate salts, oxalate salts, malonate salts, succinate salts, glutarate salts, adipate salts, tartrate salts, maleate salts, malate salts, and mandelate salts.

In the present invention, no particular limitation is imposed on the drugs. The amount of administration of drugs having sufficient bioavailability to peroral administration can be reduced by enhancing the absorption in the digestive tract, to thereby reduce side effects. A desirable pharmacological effect of drugs which have poor absorption in the digestive tract and poor bioavailability to peroral administration can be obtained through peroral administration by enhancing the absorption in the digestive tract.

The drug according to the present invention is preferably a basic compound, which refers to a compound having one or two basic groups such as an amino group or an amidino group in its chemical structure.

Examples of the drugs selected from the above-described basic compounds include aromatic amidine derivatives, i.e., compounds having an aromatic amidine structure in chemical structure thereof. Specific examples thereof include derivatives, which are described in Japanese Patent Application Laid-Open (*kokai*) No. 5-208946 and International Patent Publication No. WO96/16940, represented by formula (1): 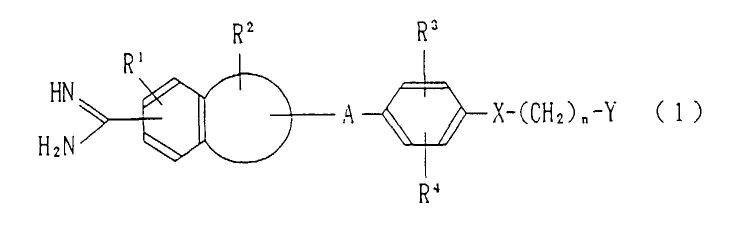 [wherein
R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group, or an alkoxycarbonylalkoxy group;
R⁴ represents a hydrogen atom, a halogen atom, an amino group, a cyano group, a nitro group, a hydroxyl group, a lower alkyl group, or a lower alkoxyl group;
n is an integer of 0 to 4;
A represents a C1-C4 alkylene group which may be substituted with one or two hydroxyalkyl groups, carboxyl groups, alkoxycarbonyl groups, carboxyalkyl groups, and alkoxycarbonylalkyl groups, or a group represented by the formula: 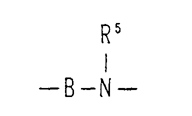 {wherein
B represents a lower alkylene group or a carbonyl group; R⁵ represents a hydrogen atom or a group represented by the formula -D-W-R⁶ (wherein D represents a group represented by the formula: 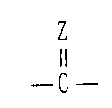 (wherein
Z represents an oxygen atom or a sulfur atom), a group represented by the formula: 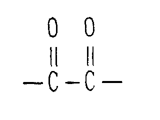 or a sulfonyl group;
W represents a single bond or a group represented by the formula -NR⁷- (wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent); and
R⁶ represents a hydroxyl group, a lower alkoxyl group, a lower alkyl group which may have a substituent, a lower aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic moiety or cyclic hydrocarbyl moiety which may have a substituent, an amino group which may have a substituent, or an aminoalkyl group which may have a substituent; and
the group represented by the formula: 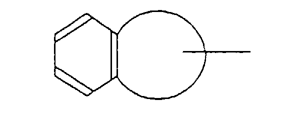 represents a group selected from among indolyl, benzofuranyl, benzothienyl, benzimidazolyl, benzoxazolyl, benzothiazolyl, naphthyl, tetrahydronaphthyl, and indanyl].

Examples of the drugs also include compounds described below.

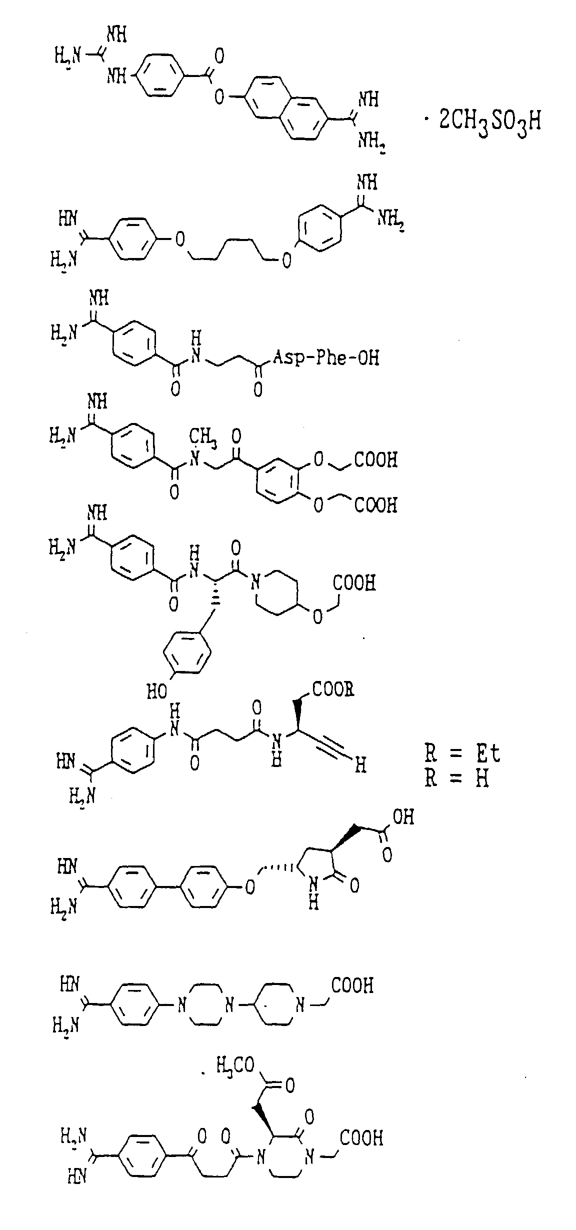

These are known compounds and may be produced through a known method.

Of the above compounds, derivatives represented by formula (1) are preferably used as the drug. Examples of the lower alkyl group in aromatic amidine derivatives according to the present invention represented by formula (1) include any of a C1-C6 linear, branched, or cyclic alkyl group. Specific examples of the lower alkyl group include a methyl group, an ethyl group, a propyl group, an isopropyl group, a butyl group, a secondary butyl group, a tertiary butyl group, a pentyl group, a hexyl group, a cyclopropyl group, a cyclobutyl group, a cyclopentyl group, and a cyclohexyl group. The lower alkyl group may have a substituent.

Examples of the group which is able to be a substituent for the lower alkyl groups include a halogen atom, a carboxyl group, a carbamoyl group, an amino group, a cyano group, a nitro group, a lower alkanoyl group, a lower alkoxyl group, a lower alkoxycarbonyl group, a mono- or di-lower alkylamino group, an aryl group, an aralkyloxy group, an aryloxy group, a mercapto group, a lower alkylthio group, a lower alkylthiocarbonyl group, a hydroxyl group, a carbamoyl group, a mono- or di-lower alkylaminocarbonyl group.

Examples of the lower alkoxyl group include a C1-C6 alkoxyl group, and examples thereof include a methoxyl group, an ethoxyl group, a propoxyl group, an isopropoxyl group, a butoxyl group, a secondary butoxyl group, and a tertiary butoxyl group.

Examples of the alkoxycarbonyl group include a methoxycarbonyl group, an ethoxycarbonyl group, a propoxycarbonyl group, and a butoxycarbonyl group.

Examples of the carboxyalkyl group include a carboxymethyl group, a carboxyethyl group, and a carboxypropyl group.

Examples of the alkoxycarbonylalkyl group include a methoxycarbonylmethyl group, an ethoxycarbonylmethyl group, a propoxycarbonylmethyl group, a methoxycarbonylethyl group, an ethoxycarbonylethyl group, a methoxycarbonylpropyl group, and an ethoxycarbonylpropyl group.

Examples of the carboxyalkoxyl group include a carboxymethoxyl group, a carboxyethoxyl group, and a carboxypropoxyl group, and examples of the alkoxycarbonylalkoxyl group include a methoxycarbonylmethoxyl group, an ethoxycarbonylmethoxyl group, a propoxycarbonylmethoxyl group, a methoxycarbonylethoxyl group, and an ethoxycarbonylethoxyl group.

Examples of the hydroxyalkyl group include a hydroxymethyl group, a hydroxyethyl group, a hydroxypropyl group, and a hydroxybutyl group. Examples of the C1-C4 alkylene group include a methylene group, an ethylene group, a trimethylene group, and a tetramethylene group.

Examples of the mono- or di-lower alkylaminocarbonyl group include as the mono-lower alkylminocarbonyl group a methylaminocarbonyl group, an ethylaminocarbonyl group, a propylaminocarbonyl group, an isopropylaminocarbonyl group, a butylaminocarbonyl group, an isobutylaminocarbonyl group, a pentylaminocarbonyl group, an isopentylaminocarbonyl group, a hexylaminocarbonyl group, and an isohexylaminocarbonyl group. Examples of the dialkylaminocarbonyl group include symmetrically lower dialkyl-substituted dialkylaminocarbonyl groups such as a dimethylaminocarbonyl group, a diethylaminocarbonyl group, a dipropylaminocarbonyl group, a dilsopropylaminocarbonyl group, a dibutylaminocarbonyl group, or a dipentylaminocarbonyl group and dialkylaminocarbonyl groups asymmetrically substituted with lower alkyl groups which are different from each other such as an ethylmethylaminocarbonyl group, a methylpropylaminocarbonyl group, an ethylpropylaminocarbonyl group, a butylmethylaminocarbonyl group, a butylethylaminocarbonyl group, or a butylpropylaminocarbonyl group.

Examples of the lower alkylsulfonyl group include a methylsulfonyl group, an ethylsulfonyl group, a propylsulfonyl group, an isopropylsulfonyl group, a butylsulfonyl group, an isobutylsulfonyl group, a pentylsulfonyl group, an isopentylsulfonyl group, a hexylsulfonyl group, and an isohexylsulfonyl group.

Examples of the mono- or di-lower alkylaminothiocarbonyl group include as the mono-lower alkyl group a methylaminothiocarbonyl group, an ethylaminothiocarbonyl group, a propylaminothiocarbonyl group, an isopropylaminothiocarbonyl group, a butylaminothiocarbonyl group, an isobutylaminothiocarbonyl group, a pentylaminothiocarbonyl group, an isopentylaminothiocarbonyl group, a hexylaminothiocarbonyl group, and an isohexylaminothiocarbonyl group. Examples of the dialkylaminothiocarbonyl group include symmetrically lower dialkyl-substituted dialkylaminothiocarbonyl groups such as a dimethylaminothiocarbonyl group, a diethylaminothiocarbonyl group, a dipropylaminothiocarbonyl group, a diisopropylaminothiocarbonyl group, a dibutylaminothiocarbonyl group, or a dipentylaminothiocarbonyl group and dialkylaminothiocarbonyl groups asymmetrically substituted with lower alkyl groups which are different from each other such as an ethylmethylaminothiocarbonyl group, a methylpropylaminothiocarbonyl group, an ethylpropylaminothiocarbonyl group, a butylmethylaminothiocarbonyl group, a butylethylaminothiocarbonyl group, or a butylpropylaminothiocarbonyl group.

Examples of the lower alkanoyl group include a formyl group, an acetyl group, a propionyl group, a butyryl group, an isobutyryl group, a valeryl group, an isovaleryl group, a pivaloyl group, and a hexanoyl group, preferably, an acetyl group, a propionyl group, and a butyryl group, more preferably an acetyl group and a propionyl group. The lower alkanoly group may have a substituent.

Examples of the group which is able to become a substituent for the lower alkanoyl groups include a halogen atom, a carboxyl group, a carbamoyl group, an amino group, a cyano group, a nitro group, a lower alkanoyl group, a lower alkoxyl group, a lower alkoxycarbonyl group, a mono- or di-lower alkylamino group, an aryl group, an aralkyloxy group, an aryloxy group, a mercapto group, a lower alkylthio group, a lower alkylthiocarbonyl group, a hydroxyl group, a carbamoyl group, a mono- or di-lower alkylaminocarbonyl group.

Examples of the aryl group, which may have a substituent, include a phenyl group, a naphthyl group, a biphenyl group, and an anthryl group.

Examples of the heteroaryl group, which may have a substituent, include a furyl group, a thienyl group, a pyrrolyl group, an imidazolyl group, a pyrazolyl group, an isothiazolyl group, an isoxazolyl group, a pyridyl group, a pyrimidinyl group, a quinolyl group, an isoquinolyl group, a quinazolinyl group, a quinolizinyl group, a quinoxalinyl group, a cinnolinyl group, a benzimidazolyl group, an imidazopyridyl group, a benzofuranyl group, a naphthyridinyl group, a 1,2-benzoisoxazolyl group, a benzoxazolyl group, a benzothiazolyl group, an oxazolopyridyl group, an isothiazolopyridyl group, and a benzothienyl group, preferably a furyl group, a thienyl group, a pyrrolyl group, an imidazolyl group, and a pyridyl group.

Examples of the group which is able to serve as a substituent for the aryl group or the heteroaryl groups include a halogen atom, a carboxyl group, an amino group, a cyano group, a nitro group, a hydroxyl group, a lower alkoxyl group, a lower alkoxycarbonyl group, a mono- or di-lower alkylamino group, a lower alkanoyl group, and a lower alkyl group which may have a substituent.

With regard to the saturated or unsaturated 5- or 6-membered heterocyclic group, heterocyclic groups having one or two hetero atoms selected from nitrogen atoms or oxygen atoms are preferred. Examples of these heterocyclic groups include pyrrolidine, piperidine, imidazoline, piperazine, tetrahydrofuran, hexahydropyrimidine, pyrrole, imidazole, pyrazine, pyrrolidinone, piperidinone, and morpholine. Examples of the saturated or unsaturated cyclic hydrocarbyl groups include a cyclopentyl group and a cyclohexyl group. Examples of the aminoalkyl group include an aminomethyl group, an aminoethyl group, and an aminopropyl group. The heterocyclic groups or the cyclic hydrocarbyl groups may have a substituent.

Examples of the group which is able to serve as a substituent for the heterocyclic groups or the cyclic hydrocarbyl groups include a lower alkyl group, a lower alkanoyl group, a carbamoyl group, a monoalkylcarbamoyl group, a dialkylcarbamoyl group, a formimidoyl group, an alkanoimidoyl group, a benzimidoyl group, a carboxyl group, an alkoxycarbonyl group, a carboxyalkyl group, an alkylcarbonylalkyl group, an aminoalkyl group, an alkanoylamino group, an alkanoylaminoalkyl group, an imino group, and an alkoxycarbonylimino group.

Examples of the group which is able to serve as a substituent for the amino moiety of the amino group or aminoalkyl group include a lower alkyl group, a pyrrolidinyl group, a pyrazyl group, a carbamoyl group, a monoalkyl carbamoyl group, a dialkylcarbamoyl group, a lower alkanoyl group, a formimidoyl group, an alkanoimidoyl group, a benzimidoyl group, and an alkoxycarbonyl group. The alkyl group, alkoxyl group, the alkanoyl group, etc. shown herein preferably contain 1-6 carbon atoms.

In formula (1), the group represented by 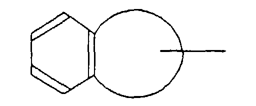 is preferably a group selected from among benzofuranyl, benzimidazolyl, indolyl, benzothienyl, benzothiazolyl, naphthyl, and tetrahydronaphthyl.

In formula (1), the saturated or unsaturated 5- or 6-membered heterocyclic group preferably contains one or two heteroatoms, selected from nitrogen atoms or oxygen atoms. A pyrrolydinyl group and a piperidinyl group are more preferred.

The aromatic amidine derivatives of formula (1) of the present invention may have asymmetric carbons, and thus there may exist optical isomers or stereoisomers based on such asymmetric carbons. Such optical isomers, stereoisomers, and mixtures thereof are also encompassed within the scope of the present invention.

In the present invention, among the compounds of formula (1), the following compounds are particularly preferred:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]phenyl]-3-(5-amidinobenzo[b]thien-2-yl)propionic acid,
3-[4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid,
2-[4-[(1-acetimidoyl-4-piperidinyl]oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetimidoyl-4-piperidyl)oxy]phenyl]-N-[(7-amidino-2-naphthyl)methyl]sulfamoyl)benzoic acid,
N-[4-[(1-acetimidoyl-4-piperidyl)oxy]phenyl)-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine,
   and N-[N-4-[(1-acetimidoyl-4-piperidyl)oxy]phenyl]-N-[(7-amidino-2-naphthyl)methyl]sulfamoyl]glycine.

The following compounds are even more preferred:
(2S)-2-[4-[((3S)-1-acetimidoyl-3-pyrrolidinyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
(+)-2-[4-[((2S)-1-acetimidoyl-2-pyrrolidinyl)methoxy]phenyl]-3-(5-amidinobenzo[b]thien-2-yl)propionic acid,
ethyl N-[N-[4-[(1-acetimidoyl-4-piperidyl)oxy]phenyl]-N-[(7-amidino-2-naphthyl)methyl]sulfamoyl]glycinate, and
N-[N-4-[(1-acetimidoyl-4-piperidyl)oxy]phenyl]-N-[(7-amidino-2-naphthyl)methyl]sulfamoyl]glycine.

Specifically, preferred compounds include:
(2S)-2-[4-[((3S)-1-acetimidoyl-3-pyrrolidinyl)oxy]phenyl]-3-(7-amidino-2-naphthyl)propionic acid hydrochloride pentahydrate,
(+)-2-[4-[(1-acetimidoyl-4-piperidyl)oxy]phenyl]-3-(7-amidino-2-naphthyl)propionic acid dihydrochloride,
(+)-2-[4-[((2S)-1-acetimidoyl-2-pyrrolidinyl)methoxy]phenyl]-3-(5-amidinobenzo[b]thien-2-yl)propionic acid dihydrochloride,
ethyl N-[N-[4-[(1-acetimidoyl-4-piperidyl)oxy]phenyl]-N-[(7-amidino-2-naphthyl)methyl]sulfamoyl glycinate dihydrochloride,
   and N-[N-4-[(1-acetimidoyl-4-piperidyl)oxy]phenyl]-N-[(7-amidino-2-naphthyl)methyl]sulfamoyl]glycine dihydrochloride.

The most preferable compound is (2S)-2-[4-[((3S)-1-acetimidoyl-3-pyrrolidinyl)oxy]phenyl]-3-(7-amidino-2-naphthyl)propionic acid hydrochloride pentahydrate.

The amount of the anion-exchange resin incorporated into the pharmaceutical composition of the present invention should be properly adjusted in accordance with the formulation which the drug and/or the composition may take. The greater the amount of the anion-exchange resin, the more improved the expected absorption-improving effect. The daily dose of the anion-exchange resin is preferably 0.001 g - 54 g, particularly preferably 0.01 g - 36 g.

In the present invention, the drug and the anion-exchange resin may be administered concurrently. Alternatively, the drug may be administered after the anion-exchange resin is administered. When the anion-exchange resin is administered first, the time between the administration of the anion-exchange resin and the administration of the drug is preferably between zero (i.e.. immediately after) and 2 hours, particularly preferably between zero and 1 hour.

The pharmaceutical composition of the present invention can take any form that allows absorption through the digestive tract. Examples of preferred forms thereof include those suitable for peroral administration; such as powder, fine granules, granules, pills, tablets, capsules, liquids, dry syrup, syrup, suspensions, and emulsions. Of these, fine granules, granules, tablets, capsules and dry syrup are particularly preferred.

The pharmaceutical composition of the present invention may be prepared according to a known method. There may be added suitable additives such as excipients, disintegrants, binders, lubricants, fluidizing agents, dispersants, suspension agents, emulsifiers, preservatives, and stabilizers.

Preferably, the formulation is constructed such that it allows the drug and the anion-exchange resin to coexist in the digestive tract, or that it allows sustained release of the drug and the anion-exchange resin, to thereby provide enhanced effects.

Examples of the formulation that allows the drug and the anion-exchange resin to coexist in the digestive tract include, in addition to ordinary formulations (such as powder and fine granules), fine particulate formulations such as microcapsules, which can encapsulate the drug and the anion-exchange resin therein; emulsions; enteric formulations which release the drug and the anion-exchange resin in the small intestine; timed-release-type or pulse-released-type formulations which are able to release the drug and the anion-exchange resin locally after a predetermined lag time; and mucosa-adhesion type formulations which adhere to the mucosa and gradually release the drug and the anion-exchange resin.

Examples of the sustained release formulations which release the anion-exchange resin in a sustained manner include a multi-layered formulation which is formed of a drug layer and an anion-exchange resin layer; a dry-coated formulation in which the surface of a drug-containing core is covered by an anion-exchange resin; and a multi-step-release-type formulation containing a plurality of formulations of different release behaviors.

The present invention will be described hereunder by way of example. However, the examples provided herein should in no way be construed as limiting the present invention thereto.

### Examples

### [Comparative Example 1]

(2S)-2-[4-[((3S)-1-acetimidoyl-3-pyrrolidinyl)oxy]phenyl]-3-(7-axuidino-2-naphthyl)propionic acid hydrochloride pentahydrate (hereinafter abbreviated as compound A) was dissolved in physiological saline to thereby obtain a solution having a concentration of 1.285 mg/ml.

The saline solution (0.5 ml) of compound A was perorally administered to each of rats (SD male rats, 8 weeks old, body weight: 220-240 g, n=5) by use of a peroral probe.

Blood was collected from the jugular vein of each rat anesthetized with ether, at 0.5, 1, 2, 4, and 8 hours after administration. The collected blood was heparinized and subjected to centrifugal separation to thereby obtain plasma.

Compound A contained in the collected plasma was subjected to high performance liquid chromatography (HPLC). From the time course change of the concentration of compound A in plasma, the area under the plasma concentration-time curve (AUC) and the maximal plasma concentration (Cₘₐₓ) were obtained.

As described in Japanese Patent Application laid-Open (*kokai*) No. 5-208946, the compound A inhibits FXa and thus is useful as an anticoagulant or as a preventive and therapeutic agent for thrombosis.

### [Example 1]

Compound A was dissolved in physiological saline to thereby form a solution having a concentration of 1.285 mg/ml, and the saline solution (0.5 ml) of compound A was perorally administered to each rat by use of a peroral probe.

At the time points of 20 minutes before, immediately before, and 20 minutes after the peroral administration of saline solution of compound A, cholestyramin-suspended saline solution (100 mg/ml, 0.5 ml) was perorally administered to each rat by use of a peroral probe. In a manner similar to that in Comparative Example 1, blood was collected and compound A contained in the collected plasma was measured for determination of AUC and Cₘₐₓ.

### [Example 2]

The procedure of Example 1 was repeated except that the concentration of cholestyramin-suspended saline solution was changed from 100 mg/ml to 26.6 mg/ml, and the suspension and compound A were simultaneously and perorally administered to each rat, to thereby obtain AUC and Cₘₐₓ of compound A in plasma.

The AUC data and Cₘₐₓ data obtained from Comparative Example 1, Example 1, and Example 2 are shown in Table 1.

**Table 1**

| (Average ± S.D.) | | |
|---|---|---|
| | AUC (hr·µg/ml) | Cₘₐₓ (µg/ml) |
| Comparative Example 1 | 0.56 ± 0.15 | 0.19 ± 0.08 |
| Example 1 | 1.48 ± 0.33 | 0.46 ± 0.26 |
| Example 2 | 1.12 ± 0.41 | 0.37 ± 0.12 |

### [Comparative Example 2]

Compound A was dissolved in physiological saline to thereby form a solution having a concentration of 2.57 mg/ml.

Compound A was perorally administered to each of cynomolgus monkeys (female, body weight: 2.8-3.2 kg, n=4) by use of a catheter such that each monkey would take compound A in an amount of 2.57 mg/kg.

Blood was collected from the femoral vein of each cynomolgus monkey at 0.5, 1, 2, 3, 4, and 8 hours after administration. The collected blood was heparinized and subjected to centrifugal separation to thereby obtain plasma.

Compound A contained in the collected plasma was subjected to radioimmunoassay (RIA) and the concentration of compound A contained in the collected plasma was measured. From the time course change of the concentration of compound A in plasma, AUC and Cₘₐₓ were obtained.

### [Example 3]

Compound A and cholestyramin were dissolved and suspended in physiological saline to thereby form a solution (concentration of compound A: 2.57 mg/ml, concentration of cholestyramin: 33.3 mg/ml). The physiological saline was perorally administered to each cynomolgus monkey by use of a catheter such that the monkey would take compound A in an amount of 2.57 mg/kg of.

AUC and Cₘₐₓ were obtained after blood was collected and compound A contained in the collected plasma was measured by use of the same method as in Comparative Example 2.

### [Example 4]

Film-coated tablets each having the following composition were prepared by use of a known method, and one tablet was perorally administered to cynomolgus monkeys.

| Components | Amounts (mg) |
|---|---|
| Compound A | 7.7 |
| Cholestyramin | 100 |
| Hydroxypropylcellulose | 3.2 |
| Low-substituted hydroxypropylcellulose | 12.3 |
| Magnesium stearate | 0.6 |
| Hydroxypropylmethylcellulose 2910 | 2.5 |
| Talc | 0.4 |

In a manner similar to that described in Comparative Example 2. blood was collected and compound A contained in the collected plasma was measured for determination of AUC and Cₘₐₓ.

AUC and Cₘₐₓ obtained from Comparative Example 2, Example 3, and Example 4 are shown in Table 2.

**Table 2**

| (Average ± S.D.) | | |
|---|---|---|
| | AUC (hr·µg/ml) | Cₘₐₓ (µg/ml) |
| Comparative Example 2 | 0.48 ± 0.15 | 0.17 ± 0.08 |
| Example 3 | 0.97 ± 0.22 | 0.25 ± 0.07 |
| Example 4 | 0.85 ± 0.33 | 0.24 ± 0.06 |

### [Example 5]

A dry syrup having the following composition was prepared by use of a known method.

| Components | Amounts (mg) |
|---|---|
| Compound A | 128.5 |
| Cholestyramin | 2000 |
| Polyvinyl pyrrolidone | 65 |
| Sucrose | 227.5 |
| Carmellose-Na | 25 |
| Aspartame | 50 |
| Food yellow No. 4 | 2 |
| Orange-micron | 2 |

### Industrial Applicability

As is apparent from the results shown in Tables 1 and 2, as described above, the pharmaceutical composition of the present invention comprising a drug and an anion-exchange resin exhibits excellent absorbability by the digestive tract as compared with the case of peroral administration of the drug alone.

Thus, the anion-exchange resins are useful as low-toxic absorption enhancers for drugs.

## Claims

1. An absorption enhancer for a drug in the digestive tract, which comprises an anion-exchange resin as an active component.

2. A method for promoting absorption of a drug in the digestive tract by use of an anion-exchange resin.

3. A pharmaceutical composition comprising a drug and an anion-exchange resin.

4. A pharmaceutical composition according to Claim 3 in which the drug is a basic compound.

5. A pharmaceutical composition according to Claim 3 in which the drug is an aromatic amidine compound.

6. A pharmaceutical composition comprising an anion-exchange resin and an aromatic amidine derivative represented by the following formula (1): 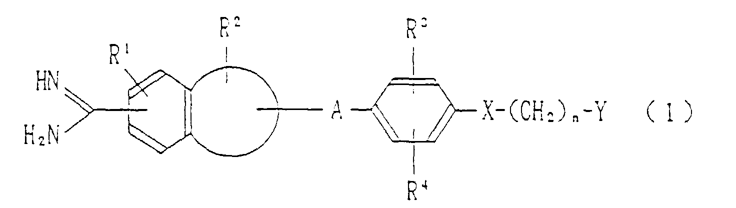 [wherein
R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group, or an alkoxycarbonylalkoxy group;
R⁴ represents a hydrogen atom, a halogen atom, an amino group, a cyano group, a nitro group, a hydroxyl group, a lower alkyl group, or a lower alkoxyl group;
n is an integer of 0 to 4;
A represents a C1-C4 alkylene group which may be have one or two substituents selected from the group consisting of hydroxyalkyl group, carboxyl group, alkoxycarbonyl group, carboxyalkyl group, and alkoxycarbonylalkyl group, or a group represented by the formula: 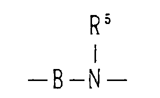 (wherein
B represents a lower alkylene group or a carbonyl group; R⁵ represents a hydrogen atom or a group represented by the formula -D-W-R⁶ (wherein D represents a group represented by the formula: 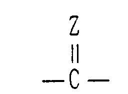 (wherein
Z represents an oxygen atom or a sulfur atom), a group represented by the formula: 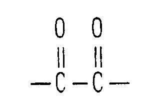 or a sulfonyl group;
W represents a single bond or a group represented by the formula -NR⁷- (wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent); and
R⁶ represents a hydroxyl group, a lower alkoxyl group, a lower alkyl group which may have a substituent, a lower aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic moiety or cyclic hydrocarbyl moiety which may have a substituent, an amino group which may have a substituent, or an aminoalkyl group which may have a substituent; and
the group represented by the formula: 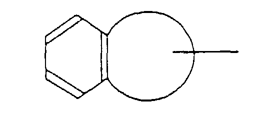 represents a group selected from among indolyl, benzofuranyl, benzothienyl, benzimidazolyl, benzoxazolyl, benzothiazolyl, naphthyl, tetrahydronaphthyl, and indanyl].

7. A pharmaceutical composition according to Claim 6, wherein the group shown by: 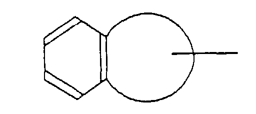 is a group selected from the group consisting of benzofuranyl, benzimidazolyl, indolyl, benzothienyl, benzothiazolyl, naphthyl, and tetrahydronaphthyl.

8. A pharmaceutical composition according to Claim 6, wherein the saturated or unsaturated 5- or 6-membered heterocyclic group in formula (1) contains, as heteroatoms, one or two nitrogen or oxygen atoms.

9. A pharmaceutical composition according to Claim 6 or 8, wherein the saturated or unsaturated 5- or 6-membered heterocyclic group in formula (1) is a pyrrolidinyl group or a piperidyl group.

10. A pharmaceutical composition according to any one of Claims 5 through 9, wherein the aromatic amidine derivative is selected from the group consisting of:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]-phenyl]-3-(5-amidinobenzo[b]thien-2-yl)propionic acid,
3-[4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid,
2-[4-[(1-acetimidoyl-4-piperidyl)oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetomidoyl-4-piperidyl)oxy]phenyl]-N-[(7-amidino-2-naphthyl)methyl]sulfamoyl]benzoic acid,
N-[4-[(1-acetimidoyl-4-piperidyl)oxy]phenyl]-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine,
and
N-[N-4-[(1-acetimidoyl-4-piperidyl)oxy]phenyl]-N-[(7-amidino-2-naphthyl)methyl]sulfamoyl]glycine.

11. A pharmaceutical composition according to any one of Claims 5 to 10, wherein the aromatic amidine derivative is selected from the group consisting of:
(2S)-2-[4-[((3S)-1-acetimidoyl-3-pyrrolidinyl)oxy]phenyl]-3-(7-amidino-2-naphthyl)propionic acid hydrochloride pentahydrate,
(+)-2-[4-[(1-acetimidoyl-4-piperidyl)oxy]phenyl]-3-(7-amidino-2-naphthyl)propionic acid dihydrochioride,
(+)-2-[4-[((2S)-1-acetimidoyl-2-pyrrolidinyl)methoxy]-phenyl]-3-(5-amidinobenzo[b]thien-2-yl)propionic acid dihydrochloride,
ethyl N-[N-[4-[(1-acetimidoyl-4-piperidyl)oxy]phenyl]-N-[(7-amidino-2-naphthyl)methyl]sulfamoyl]glycinate dihydrochloride,
and N-[N-4-[(1-acetimidoyl-4-piperidyl)oxy]phenyl]-N-[(7-amidino-2-naphthyl)methyl]sulfamoyl]glycine dihydrochloride.

12. A pharmaceutical composition containing (2S)-2-[4-[((3S)-1-acetimidoyl-3-pyrrolidinyl)oxy]phenyl]-3-(7-amidino-2-naphthyl)propionic acid hydrochloride pentahydrate and an anion-exchange resin.

13. A pharmaceutical composition according to any one of Claims 3 through 12, wherein the anion-exchange resin is selected from the group consisting of cholestyramin, colestipol hydrochloride, a compound having a structural unit represented by the following formula: 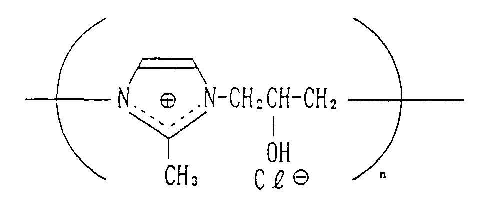 and a compound having a structural unit represented by the following formula.

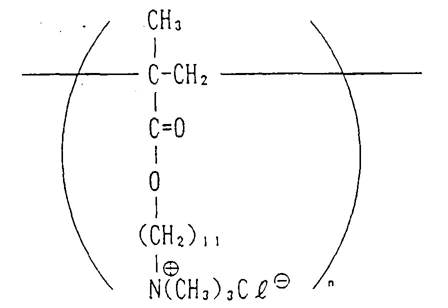

14. A pharmaceutical composition according to any one of Claims 3 to 13, wherein the anion-exchange resin is cholestyramin.

15. A pharmaceutical composition containing (2S)-2-[4-[((3S)-1-acetimidoyl-3-pyrrolidinyl)oxy]phenyl]-3-(7-amidino-2-naphthyl)propionic acid hydrochloride pentahydrate and cholestyramin.

16. A pharmaceutical composition according to any one of Claims 3 to 15, which is in the form of tablet or dry syrup.
